## Europäisches Patentamt

## European Patent Office

## Office européen des brevets

(19)

(11) Veröffentlichungsnummer: **0 340 251 B1**

(12) **EUROPÄISCHE PATENTSCHRIFT**

(45) Veröffentlichungstag der Patentschrift :
13.05.92 Patentblatt 92/20

(51) Int. Cl.⁵ : **A61M 25/00**

(21) Anmeldenummer : **88907308.6**

(22) Anmeldetag : **27.08.88**

(86) Internationale Anmeldenummer :
**PCT/EP88/00769**

(87) Internationale Veröffentlichungsnummer :
**WO 89/03701 05.05.89 Gazette 89/10**

(54) SCHLAUCH- ODER ROHRFÖRMIGER FÜHRUNGSKATHETER.

(30) Priorität : **27.10.87 DE 3736226**

(43) Veröffentlichungstag der Anmeldung :
**08.11.89 Patentblatt 89/45**

(45) Bekanntmachung des Hinweises auf die
Patenterteilung :
**13.05.92 Patentblatt 92/20**

(84) Benannte Vertragsstaaten :
**AT BE CH DE FR GB IT LI LU NL SE**

(56) Entgegenhaltungen :
**DE-A- 3 018 608**
**DE-A- 3 339 179**
**FR-A- 521 757**
**GB-A- 1 547 328**
**US-A- 4 411 055**

(73) Patentinhaber : **Schneider (Europe) AG**
**Schärenmoosstrasse 115**
**CH-8052 Zürich (CH)**

(72) Erfinder : **HARMJANZ, Dietrich**
**Fuchswinkel 20**
**W-3101 Gross Hehlen (DE)**

(74) Vertreter : **Leine, Sigurd, Dipl.-Ing. et al**
**LEINE & KÖNIG Patentanwälte**
**Burckhardtstrasse 1**
**W-3000 Hannover 1 (DE)**

EP 0 340 251 B1

Jouve, 18, rue Saint-Denis, 75001 PARIS

## Beschreibung

Die Erfindung betrifft einen schlauch- oder rohrförmigen Führungskatheter der im Oberbegriff des Anspruchs 1 genannten Art zur Aufnahme und Führung eines Ballonkatheters zur Erweiterung von Koronararterien des Herzens.

Durch die Druckschrift der Firma Schneider Medintag, Zürich, "Führungskatheter mit atraumatischer Spitze" Typ "SOFT TOUCH" ist ein Schlauch- oder Führungskatheter der betreffenden Art bekannt, dessen Ende einen ringförmigen Rand aufweist, der im wesentlichen axial zur Katheterachse verläuft. Der Katheter hat im unmittelbaren Bereich seines Endes eine mehr oder weniger gekrümmte Form, um die Einführung des Endes von der Aorta aus in die Mündung einer Koronararterie zu erleichtern oder doch wenigstens zu ermöglichen. Der Führungskatheter wird von Hand aus so bewegt, daß er mit seinem Ende in eine Koronararterie eintritt und dort verbleibt, wonach ein Führungsdraht durch den Führungskatheter hindurch in die Koronararterie eingeführt und dann über den Führungsdraht und durch den Führungskatheter ein Ballonkatheter eingeführt wird, bis der Ballon in den Bereich einer Verengung der Koronararterie gelangt, wo der in seinem Durchmesser begrenzte Ballon aufgepumpt und so die verengte Stelle der Koronararterie erweitert und gedehnt wird.

Der Durchmesser des Führungskatheters ist relativ groß, um bei ausreichender Wandungsstärke die Hindurchführung eines Ballonkatheters zu ermöglichen. Dieser verhältnismäßig große Durchmesser des Führungskatheters führt in vielen Fällen dazu, daß er die Eintrittsöffnung der Koronararterie dicht verschließt, so daß die Blutversorgung der Koronararterie aufgehoben wird. Hierdurch wird der Herzmuskel ischämisch mit der Gefahr abzusterben.

Zur Vermeidung dieses Nachteils ist in der genannten Firmendruckschrift auch ein Führungskatheter angegeben, der etwas entfernt von seinem Ende Seitenlöcher aufweist, durch die Blut aus der Aorta in den Führungskatheter eintreten und in die Koronararterie weiterströmen soll. Liegen diese seitlichen Öffnungen zu nahe am vorderen Rand des Führungskatheters, so besteht die Gefahr, daß sich die Öffnungen im Bereich der Koronararterie befinden und somit durch deren Wandung verschlossen werden, so daß sie ihren Zweck nicht erfüllen können. Liegen die seitlichen Löcher weiter entfernt vom vorderen Rand des Endes des Führungskatheters, so kann zwar Blut einströmen, jedoch besteht dann der Nachteil, daß Kontrastmittel, das zur Röntgendarstellung der Koronararterie durch den Führungskatheter in die Koronararterie eingespritzt werden soll, zu einem großen Teil aus den seitlichen Löchern ausströmt, für die Darstellung der Koronararterie verloren geht und durch Eintreten in die Aorta die Röntgendarstellung verhindert oder zumindest verschlechtert. Außerdem besteht der Nachteil, daß die seitlichen Löcher dazu neigen, durch Blutgerinnsel verstopft zu werden.

Der Erfindung liegt die Aufgabe zugrunde, einen Führungskatheter der betreffenden Art anzugeben, der die geschilderten Nachteile nicht aufweist, also eine Unterbrechung der Blutversorgung der Koronararterie und das Austreten von Kontrastmittel aus dem Führungskatheter in die Aorta vermeidet und gleichzeitig die gewünschte Führungsfunktion nicht verliert.

Die der Erfindung zugrundeliegende Aufgabe wird durch die im Kennzeichen des Anspruchs 1 angegebene Lehre gelöst.

Der Grundgedanke dieser Lehre besteht darin, die Wandung des Führungskatheters im vorderen Bereich mit einem solchen Sackschlitz zu versehen, der z. B. durch einfaches seitliches Wegschneiden des Katheters erzeugt werden kann. Wird ein solcher Führungskatheter nur so weit in die Eintrittsöffnung einer Koronararterie eingeschoben, daß das hintere Ende der Ausnehmung bzw. des Sackschlitzes noch im Bereich der Aorta liegt, so kann Blut aus der Aorta in den Katheter und durch diesen in die Koronararterie fließen. Bereits in dieser Einführungslage kann der Führkungskatheter seine Führungsfunktion voll erfüllen. Soll Kontrastmittel eingeleitet werden, so läßt sich der Führungskather kurzzeitig weiter vorschieben, so daß die Ausnehmung bzw. der Sackschlitz über seine volle Länge im Bereich der Eintrittsöffnung der Koronararterie liegt und daher durch deren Wandung weitgehend verschlossen ist. Dieses Vorschieben kann kurzzeitig erfolgen, wenn Kontrastmittel eingespritzt wird. Dann kann der Führungskatheter wieder etwas zurückgezogen werden, so daß wieder Blut in der zuvor beschriebenen Weise durch die Ausnehmung in der Wandung des Führungskatheters in die Koronararterie eintreten kann.

Die erfindungsgemäße Ausnehmung kann in Längsrichtung des Katheters eine gleiche Breite haben, die aber insgesamt einen wesentlich größeren Strömungsquerschnitt hat als die bisher bekannten, seitlichen Öffnungen. Damit ist die Gefahr der Verstopfung durch Blut verringert. Es ist aber auch zweckmäßig, daß die Breite der Ausnehmung zum vorderen Rand des Katheters hin zunimmt. Durch verschieden weites Einschieben des Endes des Führungskatheters ist somit der Querschnitt variabel, durch den Blut aus der Aorta in die Koronararterie strömen kann.

Eine andere Weiterbildung der Erfindung besteht darin, daß der Katheter im Bereich der Ausnehmung in an sich bekannter Weise aus einem nachgiebigeren Material besteht als in den übrigen Bereichen. Durch diese Nachgiebigkeit soll bei dem bekannten Katheter eine seitliche Biegsamkeit und eine Verbesserung der Einführung in die Eintrittsöffnung einer Koronararterie erzielt werden. Der Querschnitt dieses bekannten Katheters ändert sich dabei nicht. Im Falle des erfindungsgemäßen Katheters ermöglicht die Ausnehmung eine Querschnittsverengung des Katheters, so daß dieser je nach Form, Breite und Länge der Ausnehmung eine mehr oder weniger verengte oder gar konische Form erhält, wodurch die Ausführung in die Öffnungsmündung der Koronararterie zusätzlich erleichtert wird. Durch die Erfindung wird also nicht nur der Blutstrom sichergestellt und das Austreten von Kontrastmittel vermieden, sondern zusätzlich noch die Einführung in die Eintrittsöffnung der Koronararterie erleichtert.

Anhand der Zeichnung soll die Erfindung näher erläutert werden.

Fig. 1 zeigt ein Ausführungsbeispiel eines Führungskatheters gemäß der Erfindung in kleiner Darstellung,

Fig. 2 zeigt vergrößert das Ende des Führungskatheters gemäß Fig. 1 im Bereich einer Aorta und Koronararterie,

Fig. 3 zeigt das äußere Ende des Katheters gemäß Fig. 1 in starker Vergrößerung in Richtung auf eine Ausnehmung gemäß der Erfindung,

Fig. 4 ist eine Seitenansicht der Fig. 3,

Fig. 5 zeigt eine Abwandlung in der Darstellung gemäß Fig. 3 und

Fig. 6 ist eine Seitenansicht der Fig. 5.

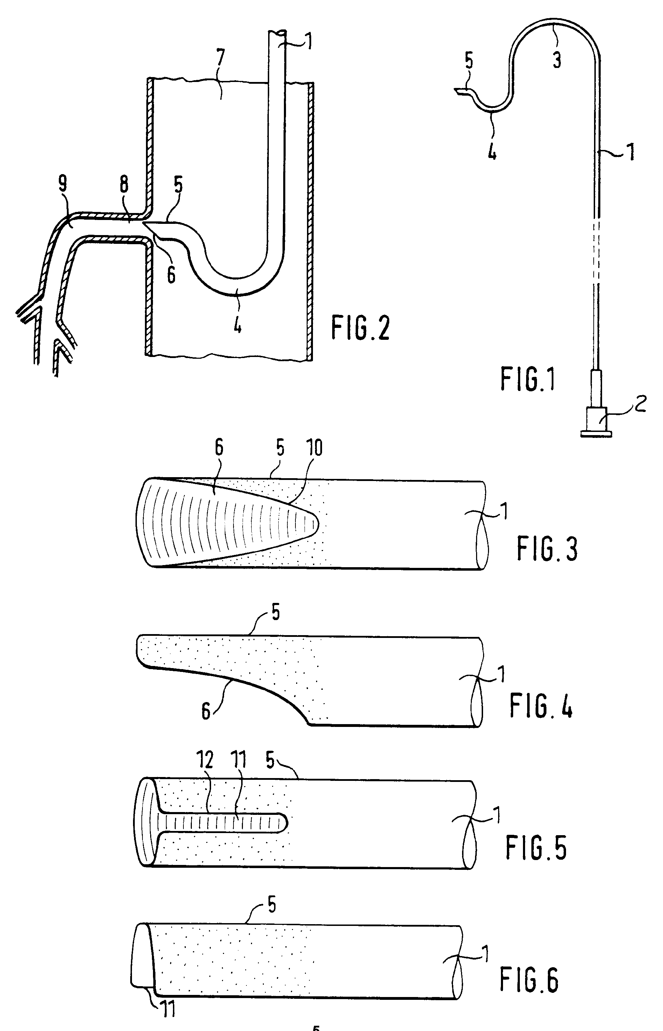

Fig. 1 zeigt einen Führungskatheter 1, der an seinem hinteren Ende mit einem Anschlußstück 2 versehen ist und in seinem vorderen Bereich einmal in einem großen Bogen 3 in der einen Richtung und einmal in einem kleineren Bogen 4 in eine andere Richtung gekrümmt ist.

Insbesondere aus Fig. 2 ist zu ersehen, daß das äußerste Ende 5 eine durch schräges Wegschneiden gebildet Ausnehmung 6 aufweist.

Der Führungskatheter 1 befindet sich in einer Aorta 7 und ragt mit seinem äußeren Ende 5 teilweise in eine Eintrittsöffnung 8 einer Koronararterie 9. In dieser Lage hat die Ausnehmung 6 einen großen Zugangsquerschnitt von der Aorta 7 her, so daß Blut in das Innere des rohrförmigen Führungskatheters und weiter in die Eintrittsöffnung 8 und die Koronararterie 9 strömen kann. In dieser gezeigten Lage kann bereits ein Führungsdraht und auch ein Ballonkatheter eingeführt werden.

Soll Kontrastmittel eingespritzt werden, so kann das Ende 5 des Führungskatheters 1 durch entsprechende Manipulation so in der Zeichnung nach links und damit in die Eintrittsöffnung 8 der Koronararterie 9 eingeschoben werden, daß die Ausnehmung 6 vollständig im Bereich der Eintrittsöffnung 8 liegt. Dieses Vorschieben erfolgt nur kurzzeitig während des Einspritzens von Kontrastmittel. Danach wird das Ende 5 sofort wieder in die in Fig. 2 gezeigte Lage zurückgezogen, so daß wieder Blut ein- und durchströmen kann und die Blutversorgung der Koronararterie hergestellt ist.

Fig. 3 zeigt das äußere Ende 5 des Führungskatheters 1 im Bereich der Ausnehmung 6 mit Blickrichtung auf diese Ausnehmung 6. Fig. 4 ist eine Seitenansicht von Fig. 3. Es ist zu ersehen, daß die Wandung des Endes 5 nicht gerade weggeschnitten ist, sondern in Form einer S-Kurve, sodaß in der Draufsicht gemäß Fig. 3 der Rand 10 der Ausnehmung 6 ungefähr eine Glockenkurve bildet. Das Ende 5 ist aus einem Material gebildet, das nachgiebiger ist als das Material des übrigen Führungskatheters 1. Dieses nachgiebige Material ist durch Punktierung angedeutet.

Die Fig. 5 und 6 entsprechen im wesentlichen den Fig. 3 und 4. Gleiche Teile sind mit gleichen Bezugsziffern versehen. Ein Unterschied besteht lediglich darin, daß eine Ausnehmung 10 im wesentlichen parallele Ränder 12 hat, so daß ein gerader Schlitz gebildet ist, der zum vorderen Rand des Endes 5 hin offen ist.

Durch die erfindungsgemäße Ausbildung des Endes des Führungskatheters wird ein total dichter Abschluß des Führungskatheters am Eingang der Koronararterie vermieden. Sollte es dennoch zu einem dichten Abschluß kommen, so wird dies richtig durch Druckabfall am Manometer angezeigt. Mit einem Zurückziehen des Katheters läßt sich der Fluß sofort wiederherstellen, ohne daß die Position des Führungskatheters verlorengeht.

Die Gefahr einer Gerinnselbildung ist bei dem erfindungsgemäßen Katheter geringer als bei dem bekannten Führungskatheter mit Seitenlöchern, da der Führungskatheter nur diese eine große Öffnung hat, die wie üblich gespült wird. Weiterhin geht kein Kontrastmittel in die Aorta verloren, da man durch Verschieben die Öffnungsfläche verkleinern kann im Gegensatz zu Seitenlöchern, die nicht regulierbar sind. Es ist ein sicheres Arbeiten mit einer Minderung der Gefahr einer Ischämie und einer Optimierung der Darstellung der Koronararterien durch Kontrastmittel gewährleistet.

## Patentansprüche

1. Schlauch- oder rohrförmiger Führungskatheter zur Aufnahme und Führung eines Ballonkatheters zur Erweiterung von Koronararterien des Herzens, **dadurch gekennzeichnet,** daß die Wandung des Katheters (1) im Bereich des vorderen Endes (5) des Katheters (1) vom vorderen Rand aus nach hinten über einen Teil des Umfangs zurückspringt, derart, daß in der Wandung eine sich vom vorderen Rand aus nach hinten erstreckende Ausnehmung (6) gebildet ist.

2. Führungskatheter nach Anspruch 1, **dadurch gekennzeichnet,** daß die Ausnehmung (1) in Längsrichtung des Katheters (1) eine gleiche Breite hat.

3. Führungskatheter nach Anspruch 1, **dadurch gekennzeichnet,** daß die Breite der Ausnehmung (6) zum vorderen Ende (5) des Katheters (1) hin zunimmt.

4. Führungskatheter nach Anspruch 3, **dadurch gekennzeichnet,** daß die Breite der Ausnehmung (6) zum vorderen Ende des Katheters (1) hin zunächst stark, dann weniger und im Bereich des vorderen Randes wieder stark zunimmt, derart, daß der Rand der Ausnehmung (6) in der Abwicklung des Katheters (1) glockenförmig ist.

5. Führungskatheter nach Anspruch 1, **dadurch gekennzeichnet,** daß der Katheter (1) im Bereich der Ausnehmung (6, 11) in an sich bekannter Weise aus einem nachgiebigeren Material besteht als in den übrigen Bereichen.

## Claims

1. A guide catheter in the form of a hose or tube for receiving and guiding a balloon catheter for widening coronary arteries of the heart, **characterized in** that the wall of the catheter (1) in the region of the front end (5) of the catheter (1) recedes over part of the circumference from the front edge towards the rear in such a way that a recess (6) is formed in the wall, extending from the front edge towards the rear.

2. A guide catheter as in Claim 1, **characterized in** that the recess (1) has a constant width in the direction longitudinal to the catheter (1).

3. A guide catheter as in Claim 1, **characterized in** that the width of the recess (6) increases towards the front end (5) of the catheter (1).

4. A guide catheter as in Claim 3, **characterized in** that the width of the recess (6) first of all increases sharply towards the front end of the catheter (1), then less so, and in the region of the front edge again sharply, in such a way that the edge of the recesss (6) is bellshaped in the development of the catheter (1).

5. A guide catheter as in Claim 1, **characterized in** that in the region of the recess (6, 11) the catheter (1) in a manner in itself known consists of a more flexible material than in the other regions.

## Revendications

1. Cathéter de guidage en forme de tuyau ou de tube pour recevoir et guider un cathéter-ballon pour la dilatation d'artères coronariennes du coeur, caractérisé en ce que la paroi du cathéter (1) revient, dans la région de l'extrémité antérieure (5) du cathéter (1), depuis le bord antérieur vers l'arrière sur une partie de la périphérie de telle manière qu'un évidement (6) s'étendant du bord antérieur vers l'arrière est formé dans la paroi.

2. Cathéter de guidage selon la revendication 1, caractérisé en ce que l'évidement (11) a, en direction longitudinale du cathéter (1), une largeur constante.

3. Cathéter de guidage selon la revendication 1, caractérisé en ce que la largeur de l'évidement (6) augmente en direction de l'extrémité antérieure (5) du cathéter (1).

4. Cathéter de guidage selon la revendication 3, caractérisé en ce que la largeur de l'évidement (6) augmente en direction de l'extrémité antérieure du cathéter (1), d'abord fortement, puis moins et à nouveau fortement dans la région du bord antérieur de telle sorte que le bord de l'évidement (6) est, en projection du cathéter (1), en forme de cloche.

5. Cathéter de guidage selon la revendication 1, caractérisé en ce que le cathéter (1) est constitué, de manière connue en soi, d'un matériau plus flexible dans la région de l'évidement (6, 11) que dans les autres régions.

FIG.1

FIG.2

FIG.3

FIG.4

FIG.5

FIG.6